## Europäisches Patentamt
## European Patent Office
## Office européen des brevets

(19)

(11) Numéro de publication: **0 200 245**
**B1**

(12) **FASCICULE DE BREVET EUROPEEN**

(45) Date de publication du fascicule du brevet: **19.09.90**

(51) Int. Cl.⁵: **C 12 N 11/02**

(21) Numéro de dépôt: **86200517.0**

(22) Date de dépôt: **26.03.86**

(54) **Procédé de production d'une structure poreuse comprenant une matière organique dispersée dans un liant.**

(30) Priorité: **27.03.85 LU 85822**

(43) Date de publication de la demande:
**05.11.86 Bulletin 86/45**

(45) Mention de la délivrance du brevet:
**19.09.90 Bulletin 90/38**

(84) Etats contractants désignés:
**AT BE CH DE FR GB IT LI LU NL SE**

(56) Documents cités:
**EP-A-0 043 632**
**FR-A-2 237 989**
**US-A-3 766 013**
**US-A-3 843 443**

**CHEMICAL ABSTRACTS, vol. 92, no. 13, 31 mars 1980, page 291, abrégé 106656h, Columbus, Ohio, US; M. SHULTS et al.: "Continuous in vivo glucose analysis based on immobilized enzyme bonded to derivatized teflon membrane"**

(73) Titulaire: **"Centre d'Etude de l'Energie Nucléaire", "C.E.N."**
**Rue Charles Lemaire 1**
**B-1160 Bruxelles (BE)**

(72) Inventeur: **Spaepen, Gustaaf F.J.**
**Molsebaan 75B**
**B-2480 Dessel (BE)**
Inventeur: **Blanchart, Alain P.O.**
**Postelweg 5**
**B-2490 Balen (BE)**
Inventeur: **Leynen, Martin A.C.**
**Nederlandlaan 2**
**B-2440 Geel (BE)**
Inventeur: **van der Poorten, Christian J.M.E.**
**Steenweg op Zevendonk 153**
**B-2300 Turnhout (BE)**

(74) Mandataire: **De Rycker, Rudolf, Ir. et al**
**BUREAU DE RYCKER Vereenigde Octrooibureaux Belgie N.V. Arenbergstraat 13**
**B-2000 Antwerpen (BE)**

Courier Press, Leamington Spa, England.

**Description**

La présente invention est relative à un procédé de production d'une structure poreuse comprenant au moins une matière organique bioligiquement active ou d'origine bioligique dispersée dans un liant.

Il existe plusieurs formes de réalisation de structures poreuses comprenant une matière organique dispersée dans un liant. De telles structures ont de nombreuses applications notamment dans des procédés et analyses chimiques et plus particulièrement biochimiques, dans des tests biologiques, la pharmacologie et l'industrie alimentaire.

Les procédés connus pour la production de ces structures poreuses comprennent généralement une phase de traitement à une température dépassant sensiblement la température ambiante et destructive de la matière organique ou de la liaison entre cette dernière et son support dans la structure.

Il en suit que la fixation de la matière organique sur le support est nécessairement la phase terminale de la production; la fixation est alors plus difficile à réaliser et la régularité de la distribution désirée de la matière organique à travers la structure en souffre. Surtout si la matière organique est à fixer sur un support pulvérulent à incorporer dans le liant, la fixation de la matière sur le support est plus facilement contrôlée si elle se réalise sur le support pulvérulent non encore incorporé dans le liant. Le couplage entre le support et la matière organique étant de préférence à réaliser sous forme d'une liaison covalente, il est à éviter que celle-ci doive se produire avec le support déjà incorporé.

US—A—3.766.013 qui décrit des procédés de production d'une structure poreuse comprenant un liant suivant lesquels le couplage de la matière organique, en l'occurrence une enzyme, se fait sur un support organique déjà incorporé dans un liant, en l'occurrence du polytetrafluoroéthylène, fait remarquer que le couplage peut également être réalisé avant fibrillation du liant, donc avec un support non encore incorporé dans le liant, si l'enzyme supporte la haute température, mais en pratique les enzymes, et en général les matières biologiques, à incorporer, sont détruites aux températures requises pour les autres phases du procédé de production.

L'invention a essentiellement pour but de procurer un procédé pouvant être réalisé sous des conditions telles que la matière organique, donc éventuellement la matière organique déjà fixée sur un support, puisse être incorporée dans le liant sans que cette matière et/ou sa fixation sur le support ne soient détruites.

Quoiqu'il soit question ci-dessus d'une matière organique, l'invention s'applique également à l'incorporation dans un liant de plusieurs matières organiques et plus particulièrement d'un mélange de matières organiques en proportions déterminées fixées.

L'invention a plus particulièrement pour but de procurer un procédé pour incorporer une matière organique telle que des enzymes, microorganismes ou organelles, ou un mélange de telles matières organiques en proportions déterminées fixées, sous une forme finement divisée dans une matrice poreuse d'un liant sans que cette matière organique ou ce mélange de matières organiques ne doive subir une phase de traitement humide ou une phase à température élevée qui risquent de l'abimer.

A cet effet, le procédé suivant l'invention comprend la succession des opérations suivantes:

on mélange ensemble la matière organique biologiquement active ou d'origine biologique sous forme d'une poudre et le liant pulvérulent à l'état sec pour former un mélange,

on soumet ce mélange à un traitement d'agglomération pour former des agglomérats,

on soumet les agglomérats formés à un traitement de broyage pour former des agglomérats broyés et

on presse ensemble les agglomérats broyés pour former un comprimé,

toutes ces opérations étant effectuées à une température inférieure à 340°K.

Quoique le procédé suivant l'invention s'applique également à l'incorporation d'une matière organique telle quelle ou d'un mélange de matières organiques sous une forme finement divisée dans une matrice poreuse de liant, il s'applique cependant plus particulièrement à une telle incorporation d'un support ou substrat sur lequel est fixée une substance organique active telle que des enzymes, microorganismes ou organelles ou sur lequel est fixé un mélange de telles substances organiques en proportions déterminées fixées. Il s'applique encore à l'incorporation d'un mélange de tels supports.

L'invention a notamment pour but de procurer un procédé pour incorporer au moins un support sur lequel est fixée au moins une substance organique dans une matrice poreuse d'un liant sans que cette incorporation doive comporter une phase de traitement humide ou une phase à température élevée qui risquent d'altérer la fixation de la matière organique sur le support ou d'inhiber ou de détruire cette matière.

A cet effet, dans une forme de réalisation particulière de l'invention, on fixe la matière organique sur un support et on mélange ensemble le support pulvérulent chargé de la matière organique et le liant pulvérulent à l'état sec pour former un mélange.

De préférence, on effectue toutes les opérations à température ambiante.

Dans une forme de réalisation avantageuse de l'invention on soumet le comprimé à des laminages jusqu'à obtention d'un film.

Il est à remarquer qu'un procédé comportant une succession d'opérations comparable à celle suivant l'invention est connu pour la production d'une couche d'éléctrode pour une pile à combustible. Suivant ce procédé connu de EP—A—43 632 on mélange une poudre fine inorganique conductrice d'électricité et le liant. Il a été constaté suivant l'invention que de façon étonante une succession comparable d'opérations permet également d'incorporer une matière organique dans un liant en vue de la production d'une structure poreuse destinée à des applications biologiques et analogues, sans que la matière organique soit altérée.

Il ne résulte plus particulièrement pas de EP—A—43 632 que ce procédé permet de maintenir inaltérée la fixation délicate d'une matière organique sur un support lors de l'incorporation de ce dernier sous une forme finement dispersée dans une matrice poreuse d'un liant.

L'incorporation d'une matière organique, plus particulièrement de la cellulose, dans un liant, en l'occurrence le polytetrafluoroéthylène, est connue de FR—A—2 237 989.

L'incorporation se fait cependant par voie humide, d'ailleurs en vue d'une extraction ultérieure de la matière organique dont l'altération au cours de ce procédé connu n'est donc pas à éviter.

D'autres détails et particularités de l'invention ressortiront de la description de plusieurs formes de réalisation d'un procédé de production d'une structure poreuse comportant une matière organique dispersée dans un liant suivant l'invention donnée ci-après à titre d'exemple non limitatif.

La structure à produire par le procédé suivant l'invention est en général une structure sous forme d'un film ou d'un disque, donc s'étendant essentiellement en deux dimensions, mais peut également être une structure tridimensionnelle. La structure poreuse est constituée par la dispersion de la matière organique ou d'un support pulvérulent chargé de cette matière organique au sein d'une matrice poreuse constituée par le liant. Le support susdit peut également être microporeux.

La matière organique est par exemple une protéine et plus particulièrement une enzyme. Des exemples de matières organiques se retrouvent dans US—A—3.766.013 incorporé ici par voie de référence.

D'autres exemples d'une matière organique pouvant être utilisée dans le procédé suivant l'invention sont la cellulose et les levures lyophilisées. D'autres exemples encore et notamment l'amyloglucosidase sont cités dans "Immobilized Enzymes, J. C. Johnson, Noyes Data Corporation 1979" publication incorporée ici par voie de référence.

La matière organique, notamment l'enzyme, peut être couplée à un support pulvérulent. Des exemples de tels supports se retrouvent dans US—A—3.766.013 et dans "Immobilized Enzymes", publication précitée. Le couplage entre le support pulvérulent et la matière organique est réalisé avant que le support ne soit incorporé dans la structure par le procédé suivant l'invention. Les particularités de la réaction de couplage ne font pas partie de la présente demande de brevet. La réalisation de tels couplages est notamment décrite dans "Immobilized Enzymes", publication précitée.

Il est à remarquer que le couplage de la matière organique au support est de préférence une liaison covalente et que la réaction de couplage connue comme telle est une réaction difficile et complexe. Il est important suivant l'invention que cette réaction soit réalisée sur la matière organique et le support non incorporé dans la structure. Le dosage est alors plus facilement et plus précisément contrôlé. Il est également important que le support muni de la matière organique ne doive pas être soumis par la suite à des opérations pouvant altérer la matière organique ou sa fixation sur le support telles que des phases de traitement à haute température ou humides.

Le liant doit se prêter à la formation d'une matrice poreuse. Il peut notamment être choisi parmi le polyéthylène, le polypropylène, le polychlorure de vinyle, les polyfluorures de carbone et le polytétrafluoroéthylène.

Si on désire limiter ou éviter le caractère hydrophobe du liant, la poudre constituant le liant peut être hydrophilisée préalablement aux autres opérations du procédé. Cette hydrophilisation peut consister dans un traitement par un liquide hydrophilisant connu tels que les liquides mentionnés dans US—A—4.252.878 incorporé ici par voie de référence.

Le procédé proprement dit consiste en une succession des opérations suivantes.

Dans une première opération on mélange ensemble la matière organique ou le support chargé de la matière organique et le liant pulvérulent à l'état sec pour former un mélange. La matière organique ou le support chargé de la matière organique est de préférence déjà sous une forme pulvérulente.

Toutefois l'application du procédé à des matières ou des supports chargés ne présentant pas cette forme n'est pas exclue. La matière ou le support chargé doit alors être rendu pulvérulent avant ou pendant l'opération de mélange.

Les pourcentages des constituants à mélanger dépendent de la nature du liant et de la nature du support et/ou de la matière organique, mais la quantité de liant en poids par rapport à l'ensemble du mélange demeure normalement inférieure à 30%. Le mélange est réalisé dans n'importe quel mélangeur de poudre, par exemple dans un mélangeur à billes dans lequel les billes ont une vitesse de l'ordre de 0,1 à 1 m/sec par rapport à la cuve.

Dans une seconde opération on soumet le mélange sec à un traitement d'agglomération pour former des agglomérats. Ceci est réalisé dans un broyeur à billes dans lequel les billes ont une vitesse sensiblement supérieure à celle appliquée pour former le mélange, par exemple une vitesse de l'ordre de 4 à 6 m/sec par rapport à la cuve.

Ce traitement d'agglomération peut avoir une durée de plusieurs heures. Après cette opération le broyeur comprend des agglomérats se présentant sous la forme d'une peau et ayant une surface de l'ordre de quelques $cm^2$ à quelques centaines de $cm^2$.

L'agglomeration peut également être réalisée par d'autres moyens que par un broyeur à billes. Il suffit en général que le mélange soit pétri, c'est-à-dire soumis à une succession d'opérations assez violentes de déformation et de cisaillement.

Dans une troisième opération les agglomérats sont soumis à un traitement de broyage pour former des agglomérats broyés, par exemple dans un mixer ou dans un moulin du genre moulin à café.

Dans une quatrième opération les agglomérats broyés ou leur fraction utile, par exemle la fraction traversant un crible de 0,5 mm, sont comprimés dans une presse jusqu'à une épaisseur de quelques centimètres.

Dans une cinquième opération facultative le comprimé est soumis à des laminages jusqu'à ce que son épaisseur soit réduite dans un rapport de 1/10 à 1/50, Les pas de laminage qui se succèdent sont effectués sur un comprimé déplacé de 90°.

Toutes les opérations se font à température ambiante et de toute façon à une température inférieure à 340°K.

### Exemple I

20 grammes de poudre très fine d'alumine est activée par lavage dans 4M HCl d'abord, puis dans 0,02 M acétate (tampon, pH = 4,2). La poudre est dispersée en une solution de 0,02 M acétate (tampon, pH = 5) et exposée à un colorant pendant 20 minutes. Après lavage dans 0,02 M acétate (pH = 4,2) la poudre est mélangée avec 100 ml de solution aqueuse d'amyloglucosidase (90 unités) pendant 8 hrs. (comme il est décrit à la page 17 de Immobilized Enzymes, publication précitée). Ainsi une poudre est obtenue qui consiste d'un substrat d'alumine couvert d'amyloglucosidase adsorbée.

Des quantités égales de ce substrat sur lequel cette enzyme est adsorbée et de poudre de polytétrafluoroéthylène (vendu dans le commerce sous la marque Teflon 10 N) sont introduites dans un broyeur à billes de laboratoire.

4 billes en acier inoxydable d'un diamètre de 30 mm sont également introduites dans le broyeur.

Après 2 heures de rotation le broyeur est ouvert;

ses parois sont recouvertes d'une "peau" formée par le polytétrafluoroéthylène et le substrat sur lequel l'enzyme est adsorbée.

La peau est prélevée et broyée dans un mixer de cuisine.

20 grammes sont prélevés et introduits dans une presse au moyen de laquelle on forme un comprimé de 3,5 × 70 × 70 mm$^3$.

Le comprimé est laminé à froid jusqu'à l'obtention d'une feuille de 0,2 mm d'épaisseur et de 510 × 135 mm$^2$ de surface.

### Exemple II

50 grammes de levures lyophilisées et 50 grammes de poudre de polytetrafluoroéthylène sont introduites dans un broyeur à billes de laboratoire et on procède ensuite comme dans l'exemple I jusqu'à l'obtention d'une feuille.

A partir de cette feuille on estampe des disques d'un diamètre de 5 mm pouvant par exemple être utilisés dans la production d'éthanol.

### Exemple III

50 grammes de poudre de polytétrafluoroéthylène (vendu dans le commerce sous la marque Teflon 10 N) et 50 grammes de poudre de cellulose sont introduits dans un broyeur à billes de laboratoire.

4 billes en acier inoxydable d'un diamètre de 30 mm sont également introduites dans le broyeur.

Après 2 heures de rotation le broyeur est ouvert; ses parois sont recouvertes d'une "peau" formée par la cellulose et le polytétrafluoroéthylène.

La peau est prélevée et broyée dans un mixer de cuisine.

20 grammes sont prélevés et introduits dans une presse au moyen de laquelle on forme un comprimé de 3,5 × 70 × 70 mm$^3$.

Le comprimé est laminé à froid jusqu'à l'obtention d'une feuille de 0,2 mm d'épaisseur et de 510 × 135 mm$^2$ de surface.

Cette feuille présente 9,5 mg.cm$^{-2}$ de polytétrafluoroéthylène et de cellulose.

Après trempage dans l'eau on constate qu'après 12 heures la feuille a adsorbé 20% de son volume en eau.

### Exemple IV

On travaille suivant l'exemple III mais la poudre de polytétrafluoroéthylène est hydrophilisée avant son introduction dans le broyeur.

A cet effet la poudre de polytétrafluoroéthylène est plongée dans une solution à 40% en poids du produit connu dans le commerce sous la dénomination "Zonyl FSN" et y est maintenue pendant cinq jours.

Lorsqu'un échantillon de la feuille réalisée suivant cet exemple est plongé dans l'eau on constate qu'après 30 secondes la feuille a adsorbé 50% de son volume en eau.

L'invention n'est nullement limitée aux formes de réalisation décrites ci-avant et dans le cadre de la présente demande de brevet de nombreuses modifications peuvent y être apportées.

C'est ainsi par exemple que suivant l'exemple I il peut être fait usage d'autres enzymes adsorbées sur le même ou sur un autre substrat et que le produit final n'est pas nécessairement une feuille. A partir de cette feuille on peut estamper des disques et d'autres structures poreuses peuvent être réalisées.

De même dans l'exemple II la feuille peut être maintenue comme produit final et d'autres structures poreuses peuvent être réalisées avec le liant et ces levures lyophilisées ou d'autres matières organiques.

## Revendications

1. Procédé de production d'une structure poreuse comprenant au moins une matière organique biologiquement active ou d'origine biologique dispersée dans un liant, caractérisé par la succession des opérations suivantes:

on mélange ensemble la matière organique biologiquement active ou d'origine biologique sous forme d'une poudre et le liant pulvérulent à l'état sec pour former un mélange,

on soumet ce mélange à un traitement d'agglomération pour former des agglomérats,

on soumet les agglomérats formés à un traitement de broyage pour former des agglomérats broyés et

on presse ensemble les agglomérats broyés pour former un comprimé, toutes ces opérations étant effectuées à une température inférieure à 340 degrés Kelvin.

2. Procédé suivant la revendication précédente, caractérisé en ce qu'on fixe la matière organique sur un support et qu'on mélange ensemble le support pulvérulent chargé de la matière organique et le liant pulvérulent à l'état sec pour former un mélange.

3. Procédé suivant la revendication précédente, caractérisé en ce qu'on fixe la matière organique sur le support pulvérulent par liaison covalente.

4. Procédé suivant l'une ou l'autre des revendications précédentes, caractérisé en ce qu'on effectue toutes les opérations à température ambiante.

5. Procédé suivant l'une ou l'autre des revendications précédentes, caractérisé en ce qu'on soumet le comprimé à des laminages jusqu'à obtention d'un film.

6. Procédé suivant l'une ou l'autre des revendications précédentes, caractérisé en ce qu'on utilise comme liant du polytetrafluoroéthylène.

7. Procédé suivant l'une ou l'autre des revendications précédentes, caractérisé en ce qu'on hydrophilise le liant pulvérulent et qu'on mélange ensemble la matière organique sous forme d'une poudre et le liant pulvérulent hydrophilisé.

8. Procédé suivant l'une ou l'autre des revendications précédentes, caractérisé en ce qu'on utilise comme matière biologiquement active une enzyme.

**Patentansprüche**

1. Ein Verfahren zur Herstellung einer porösen Struktur, die mindestens aus einer biologisch aktiven organischen Substanz oder einer Substanz biologischen Ursprungs, dispergiert in einem Bindemittel, besteht, gekennzeichnet dadurch, daß es sich aus folgenden, der Reihe nach auszuführenden Arbeitsschritten zusammensetzt.

man mischt die biologisch aktive organische Substanz oder die Substanz biologischen Ursprungs in Pulverform und das fein pulverisierte Bindemittel in trockenem Zustand zu einer Mixtur zusammen;

man unterzieht diese Mixtur einer Agglomerationsbehandlung, um Agglomerate zu bilden;

man unterzieht die gebildeten Agglomerate einer Zerkleinerungsbehandlung, um zerkleinerte Agglomerate zu bilden und

man drückt die zerkleinerten Agglomerate zu einem Preßling zusammen, wobei alle diese Arbeitsschritte bei einer Temperatur unter 340 Grad Kelvin ausgeführt werden.

2. Das Verfahren gemäß Anspruch 1, gekennzeichnet dadurch, daß man die organische Substanz auf einem Substrat fixiert und daß man das mit der organischen Substanz geladene feinpulverige Substrat und das feinpulverige Bindemittel in trockenem Zustand zu einer Mixtur mischt.

3. Das Verfahren gemäß Anspruch 2, gekennzeichnet dadurch, daß man die organische Substanz durch kovalente Bindung auf dem Substrat fixiert.

4. Das Verfahren gemäß einem der vorgenannten Ansprüche, gekennzeichnet dadurch, daß man alle Arbeitsvorgänge bei Umgebungstemperatur ausführt.

5. Das Verfahren gemäß einem der vorgenannten Ansprüche, gekennzeichnet dadurch, daß man den Preßling Walzungen aussetzt, bis man einen Film erhält.

6. Das Verfahren gemäß einem der vorgenannten Ansprüche, gekennzeichnet dadurch, daß man als Bindemittel Polytetrafluorethylen verwendet.

7. Das Verfahren gemäß einem der vorgenannten Ansprüche, gekennzeichnet dadurch, daß man das feinpulverige Bindemittel hydrophilisiert und daß man die organische Substanz in Pulverform und das feinpulverige hydrophilisierte Bindemittel zusammenmischt.

8. Das Verfahren gemäß einem der vorgenannten Ansprüche, gekennzeichnet dadurch, daß man als biologisch aktive Substanz ein Enzym verwendet.

**Claims**

1. A process for producing a porous structure, containing as a minimum an organic material, biologically active or of biological origin, dispersed in a binder, characterized by a sequence of operations as follows:

the organic material, biologically active or of biological origin, in powder form, and the pulverulent binder in the dry state are mixed together to form a mixture;

this mixture is subjected to an agglomeration treatment in order to form agglomerates;

the agglomerates thus formed are subjected to a crushing treatment in order to form crushed agglomerates and

the crushed agglomerates are compressed together in order to form a tablet, all these operations being performed at a temperature below 340° Kelvin.

2. A process according to the preceding claim, characterized in that the organic material is fixed on a support and the pulverulent support filled with the organic material and the pulverulent binder in the dry state are mixed together to form a mixture.

3. A process according to the preceding claim, characterized in that the organic material is fixed on the pulverulent support by a covalent bond.

4. A process according to one or other of the preceding claims, characterized in that all operations are performed at ambient temperature.

5. A process according to one or other of the preceding claims, characterized in that the tablet is subjected to rolling until a film is obtained.

6. A process according to one or other of the preceding claims, characterized in that polytetrafluoroethylene is used as a binder.

7. A process according to one or other of the preceding claims, characterized in that the pulverulent binder is hydrophilized and the organic material in powder form and the hydrophilized binder are mixed together.

8. A process according to one or other of the preceding claims, characterized in that an enzyme is used as a biologically active material.